(19)

Europäisches
Patentamt
European
Patent Office
Office européen
des brevets

(11) **EP 4 313 916 B1**

(12) **EUROPEAN PATENT SPECIFICATION**

(45) Date of publication and mention
of the grant of the patent:
**11.12.2024 Bulletin 2024/50**

(21) Application number: **22711100.2**

(22) Date of filing: **17.03.2022**

(51) International Patent Classification (IPC):
***C07C 1/12*** *(2006.01)*    ***C07C 9/04*** *(2006.01)*
***C07C 9/06*** *(2006.01)*    ***C07C 11/04*** *(2006.01)*

(52) Cooperative Patent Classification (CPC):
(C-Sets available)
**C07C 1/12;** C07C 2521/06; C07C 2523/78    (Cont.)

(86) International application number:
**PCT/IB2022/052433**

(87) International publication number:
**WO 2022/200941 (29.09.2022 Gazette 2022/39)**

(54) **METHOD FOR CONVERTING CARBON DIOXIDE INTO HIGH ADDED VALUE CHEMICAL COMPOUNDS THROUGH A MECHANOCHEMICAL PROCESS UNDER CONTINUOUS GAS FLOW CONDITIONS**

VERFAHREN ZUR UMWANDLUNG VON KOHLENDIOXID IN CHEMISCHE VERBINDUNGEN MIT HOHEM ZUSATZWERT DURCH EINEN MECHANOCHEMISCHEN PROZESS UNTER KONTINUIERLICHEN GASFLUSSBEDINGUNGEN

PROCÉDÉ DE CONVERSION DE DIOXYDE DE CARBONE EN COMPOSÉS CHIMIQUES À HAUTE VALEUR AJOUTÉE PAR LE BIAIS D'UN PROCÉDÉ MÉCANOCHIMIQUE DANS DES CONDITIONS D'ÉCOULEMENT GAZEUX CONTINU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2021 IT 202100007091**

(43) Date of publication of application:
**07.02.2024 Bulletin 2024/06**

(73) Proprietor: **Universita' Degli Studi Di Sassari
07100 Sassari (IT)**

(72) Inventors:
• **TARAS, Alessandro
07100 Sassari (SS) (IT)**
• **FARINA, Valeria
07100 BOTTIDA (SS) (IT)**
• **CAPPAI, Luca
07100 SASSARI (SS) (IT)**
• **ENZO, Stefano
07100 Sassari (SS) (IT)**
• **GARRONI, Sebastiano
07100 Sassari (SS) (IT)**

• **MULAS, Gabriele
07100 Sassari (SS) (IT)**

(74) Representative: **Metroconsult Srl
Foro Bonaparte 51
20121 Milano (IT)**

(56) References cited:
**WO-A1-01/34538**

• FABIAN M ET AL: "The influence of attrition milling on carbon dioxide sequestration on magnesium-iron silicate", MINERALS ENGINEERING, ELSEVIER, AMSTERDAM, NL, vol. 23, no. 8, 1 July 2010 (2010-07-01), pages 616 - 620, XP027053941, ISSN: 0892-6875, [retrieved on 20100518]

**(Cont. next page)**

(52) Cooperative Patent Classification (CPC): (Cont.)

C-Sets
**C07C 1/12, C07C 9/04;**
**C07C 1/12, C07C 9/06;**
**C07C 1/12, C07C 11/04**

## Description

## Technical Field

The present invention relates to a method for converting carbon dioxide ($CO_2$) into high added value chemical compounds under continuous gas flow conditions. In particular, said method converts $CO_2$ into a mixture of high added value chemical compounds comprising low molecular weight hydrocarbons, mainly methane, ethylene and ethane, along with solid products of mineral carbonation, mainly Mg and Fe carbonates. Such $CO_2$ conversion is achieved through a mechanochemical process.

## Background Art and Technical Problem

Carbon dioxide ($CO_2$) is a greenhouse gas which is released into the atmosphere as a consequence of natural phenomena (such as, for example, volcanic eruptions, cellular respiration, etc.) and human activities (such as, for example, processes of combustion of organic materials of fossil and non-fossil origin (wood, oil, petrol, diesel fuel, gas, biomass, etc.), deforestation activities, etc.), the concentration of which (> 400 ppm since 2015) has exceeded by 45% the maximum historical values of the pre-industrial age. [1] [2]

Among the direct consequences of this increased $CO_2$ concentration, a higher mean global temperature, the desertification of increasingly large areas, and the occurrence of "extreme" and potentially catastrophic climatic events are only some of the most important current environment-related issues on a global scale. Mitigating such effects requires timely interventions and positive actions by all industrialized countries.[3] The carbon dioxide emission targets set by the Kyoto protocol in 1997, and then by the Paris agreement in 2015, in line with the targets of the later EU framework programmes for research and innovation (including Horizon 2020, Horizon Europe), have led the scientific community to studying new energy sources having a low environmental impact and developing new technologies and processes aimed at reducing the levels of carbon dioxide released into the atmosphere. Much attention is now being paid to reducing the use of fossil fuels, developing processes allowing for large-scale exploitation of renewable, or carbon-free, energy sources, and setting up processes and technologies for reducing the $CO_2$ concentration in the atmosphere. In this regard, the acronym **CCUS** (Carbon Capture, Utilization and Storage) refers in the literature to various technologies currently under development, but still far from possible large-scale application, which are devoted to reducing the atmospheric $CO_2$ concentration by capturing and storing $CO_2$ and transforming the captured carbon dioxide into products having a high commercial value.[4]

In particular, much attention has been paid to Carbon Capture and Storage (**CCS**) procedures in recent years, with the goal of achieving, in the short term, a considerable reduction in carbon dioxide emissions by means of mineral carbonation processes occurring in geologically stable underground basins; however, the feasibility of such a large-scale process is currently limited by several factors, including, for example, the high costs to be incurred for sequestration, transportation and the energy necessary for injecting CO2-containing exhaust gas into the subsoil.[5] On the other hand, the set of utilization processes (**CCU**) represents a virtuous way of controlling $CO_2$ concentration thanks to the possible creation of a so-called "zero carbon dioxide emission" scenario, since abundant $CO_2$ can be used as a raw material for the creation of a virtuous closed cycle, wherein the generated gas is transformed into valuable chemical compounds while at the same time balancing the atmospheric $CO_2$ levels.

In this latter respect, one aspect of exceptional interest from an application viewpoint concerns the experimental conditions necessary for activating the chemical processes for converting $CO_2$ into basic fuels and chemical products: literature data show that the activation and transformation of carbon dioxide ($CO_2$) require severe experimental conditions to overcome the slow reaction kinetics and the high energy barrier due to its thermodynamic stability ($\Delta_f G^0$ $CO_2$ = -394.36 kJ mol$^{-1}$). In general, the conversion of carbon dioxide into basic fuels and chemical substances can be exemplified by means of redox processes occurring in the presence of molecular hydrogen. It should be taken into account that hydrogen, in its turn, has long been the subject of research for potential energy vectors alternative to fossil fuels, and the search for novel processes for producing it is still underway. [6] The modes of $CO_2$ activation reported in the scientific literature refer to processes occurring in the presence of catalysts and promoted thermally or, to a limited extent, photochemically or electrochemically. [7] Catalytic processes play a crucial role in the chemical industry and will be the key for all future scenarios aimed at implementing sustainable cycles; catalysts are, in fact, compounds capable of providing a reaction path that is alternative to the original reaction path and characterized by a significantly lower activation energy barrier.

The study of transformations of natural silicates with high Fe and Mg content like, for example, Olivine, which is a mineral widely available on the earth's crust[8] that has a high potential for interaction with carbon dioxide[9], can be considered to be related to both of the above-mentioned lines of intervention (i.e. CCS and CCU) . In particular pressure and temperature conditions, Olivine can, in fact, react with water ($H_2O$) to give the mineral known as serpentine ($Mg_3Si_2O_5(OH)_4$) plus a mixed oxide of Fe2+/Fe3+ and molecular $H_2$. This process, known as serpentinization, leads to molecular $H_2$ and partial oxidation of Fe2+ and Fe3+ into magnetite oxide. In the presence of carbon dioxide, the molecular hydrogen thus formed can, through the Fischer-Tropsch process, form light hydrocarbons, including methane, or bring about $CO_2$ mineral carbonation processes. The reaction scheme can be summarized as follows:

$$\alpha_1(Fe_{0,1}Mg_{0,9})SiO_4 \cdot nH_2O \cdot nCO_2 \longrightarrow \alpha_2 Mg_3 Si_2 O_5(OH) + \alpha_3 Fe_3 O_4 + nH_{2\uparrow} + \alpha_4 MgCO_3 + nCH_{4\uparrow} + minerals$$

In the above scheme, $\alpha_1 \longrightarrow \alpha_4$ are the stoichiometric coefficients of the phases that participate in the process. During this process, it is possible to observe both "precipitation" of $CO_2$ as solid carbonate and its transformation into methane and other low molecular weight hydrocarbons, made possible by the presence of molecular hydrogen ($H_2$) produced by the dissociation of $H_2O$ occurring during the first step of the process. It should be noted that such processes already occur in nature, mainly in ocean floors, although with extremely slow reaction kinetics on a geological time scale, and have also been reproduced in the laboratory, although in the presence of particularly high pressure and temperature conditions. [10]

Considering the conditions required for the activation of the above process by thermal means, the use of less conventional forms of energy, e.g. kinetic energy, may offer some specific solutions of interest. In particular, the study of the transformations induced in chemical systems comprising at least one solid phase by supplying mechanical energy is generally referred to as "mechanochemistry": such transformations are achieved through the use of particular apparatuses, i.e. the so-called mechanochemical reactors, which may have different construction specifications and perform different mechanical actions. For example, high-energy micromills or rotary mills provided with milling means (such as balls made of suitable materials and having variable sizes) are examples of apparatuses that are commonly employed, on a laboratory scale, for the execution of mechanochemical processes. However, other types of devices provided with milling means can be used as well for executing mechanochemical processes. The continuous repetition of processes of breaking and comminution of the solid phases involved, the renewal of the exposed surfaces of the mechanically processed phases, and the local conditions achieved often make it possible to successfully obtain chemical transformations that would not be otherwise attainable by thermal treatment. The versatility of these mechanical processes and the specific conditions that can be obtained in the interaction between solid phases and gaseous phases, as well as the wide industrial diffusion of processes involving solid phases, support the study of this mode of activation of mechanochemical processes for $CO_2$ conversion, for which, however, scientific literature data are still very scant and lack a global vision that may clarify their feasibility, specificity and possible application advantages.

As already highlighted above, aiming at contributing to mitigating climate changes by reducing $CO_2$ emissions, the Carbon Capture Storage (CCS) and the more recent Carbon Capture Utilization (CCU) strategies have drawn the attention of scientists all over the world. Mineral carbonation, also known as "mineral sequestration", is the most renowned and widespread $CO_2$ storage technique among such processes. The main advantage of mineral carbonation is the formation of stable carbonates capable of "storing" $CO_2$ for long periods, thanks to its conversion into carbonate by reaction with metal oxides. As aforementioned, Olivine and Serpentine are the most promising minerals for carbon sequestration, because of their high magnesium content. In this frame, the $CO_2$ absorption process occurring during olivine serpentinization has been studied experimentally, but literature data are not homogeneous and mostly refer to the effects of mechanical activation upon olivine, focusing mainly on its structural and superficial transformation, for the purpose of promoting $CO_2$ storage [R. A. Kleiv et al. (2006), Mechanical activation of olivine. Miner. Eng. 19, 340-347; P. Baláz et al. (2008), Structural changes in olivine (Mg,Fe)2SiO4 mechanically activated in high-energy mills. Int. J. Miner. Process. 88, 1-6; M. Fabian et al. (2010), The influence of attrition milling on carbon dioxide sequestration on magnesium-iron silicate. Miner. Eng. 23, 616-620; K. L. Sandvik et al. (2011), Mechanically activated minerals as a sink for CO2. Adv. Powder Technol. 22, 416-421; I. M. Power et al. (2013), Serpentinite carbonation for CO2 sequestration. Elements 9, 115-121; E. Turianicová et al. (2013), A comparison of the reactivity of activated and non-activated olivine with CO2. Int. J. Miner. Process. 123, 73-77; J. Li et al. (2015), Ultra-fine grinding and mechanical activation of mine waste rock using a high-speed stirred mill for mineral carbonation. Int. J. Miner. Metall. Mater. 22, 1005-1016; I. Rigopoulos et al. (2015), Enhancing the rate of ex situ mineral carbonation in dunites via ball milling. Adv. Powder Technol. 27, 360-371J. In particular, it has been demonstrated that mechanical grinding of Olivine significantly increases its ability to form iron and magnesium carbonates, making $CO_2$ capture a potential method of stable long-term storage. Mechanical activation has proved to be even more effective when the treatment occurs in the presence of liquids like water [E. Turianicová et al. (2008), A possible way to storage carbon dioxide on mechanically activated olivine (Mg, Fe)2SiO4. VI Int. Conf. Mechanochemistry Mech. Alloy. 316-319] or ethanol [I. Rigopoulos et al. (2015), Carbon dioxide storage in olivine basalts: Effect of ball milling process. Powder Technol. 273, 220-229], as a consequence of the larger surface generated in humid conditions. Such studies have not paid much attention to the chemical reduction of $CO_2$ for producing methane and light hydrocarbons.

WO 01/34538 A1 discloses a process wherein a mixture of carbon dioxide and hydrogen is reacted with a Fe-Cu-K/ $\gamma$-Al2O3 catalyst at 300°C to produce hydrocarbons.

In general, in fact, the currently available technologies that concern the transformation of $CO_2$ into methane

are mostly based on thermally activated processes or on processes that exploit the activity of special bacteria. No examples exist in the literature which refer to the transformation of $CO_2$ into hydrocarbons, such as, for example, methane and other light hydrocarbons like ethane or ethylene, through the use of mechanochemical activation methods. Also, there are no documents relating to the transformation of carbon dioxide into methane and light hydrocarbons through the use of natural minerals like Olivine, in a process exploiting a mechanical method to achieve $CO_2$ transformation.

### Drawbacks of the Prior Art

Notwithstanding the importance of the above-illustrated themes from an environmental and economical viewpoint, to the present inventors' knowledge no consolidated and commercially available technologies currently exist which are applicable at an industrial level for controlling the processes of capturing, storing and converting atmospheric $CO_2$: the extended research activity of the scientific community in this field, referable to the above-mentioned CCS (Carbon Capture and Storage) and CCU (Carbon Capture and Utilization) processes, has been mostly directed to studying, respectively, mineral carbonation processes and confinement on geological sites and conversion processes via high-temperature catalytic treatments. In this latter case, the focus is mainly on setting up complex catalytic systems, often obtained starting from precious metals and other chemical systems, via procedures referred to as fine chemistry, and such catalysts generally cannot be easily and economically produced and used on an industrial scale. Furthermore, the operating conditions required by the conversion processes, also in the presence of the above-mentioned catalytic systems, are far from standard conditions: the temperatures of the processes for transforming $CO_2$ into fuels, studied in pre-application conditions, are generally >300°C and represent themselves a source of high energy consumption and environmental pollution.

Lastly, it must be underlined that no technologies are currently available which are based on the transfer of mechanical energy for activating the above-mentioned capture, storage and utilization processes.

Another aspect must also be pointed out which has not been taken into account in previous studies, while however being connected with the experimental/actual conditions under which $CO_2$ is released into the atmosphere. When not related to natural phenomena, the processes of $CO_2$ production are connected to processes of combustion of organic materials used on an industrial scale, generally fossil fuels (but this also applies to combustion of biomass, wood, coal, etc.) such as, for example, energy production processes in thermoelectric power plants, iron and cement production, processes related to automotive systems, as well as air-conditioning processes, feeding processes, etc. The experimental conditions under which the combustion phenomena occur are far from standard (e.g. the combustion of fossil materials in the boilers of thermoelectric power plants reaches temperature values of approximately 560°C, i.e. much higher than ambient temperature, and pressure values much higher than atmospheric pressure), but the produced flow of $CO_2$ is generally released into the atmosphere at a pressure only slightly higher than atmospheric pressure and at a temperature of approximately $\leq 100$°C. Merely by way of example, the fumes exiting the post-combustion chimney in the typical conditions of a thermoelectric power plant are characterized by temperature values of approximately $\leq$ 100°C (90-95 °C) and a pressure close to atmospheric pressure (approx. 100,000 Pa), and their composition generally includes $N_2$ (approx. 75%), $O_2$ (approx. 9%), $H_2O$ (approx. 6 %), $CO_2$ (approx. 9%). Therefore, the fumes consist of a humid mixture containing excess thermal energy and kinetic energy, and are released into the atmosphere under continuous flow conditions, at a velocity of 10 to 20 m/s, and with flow rate values that depend on the size of the chimney. Conditions not too different from these can be found in other industrial chimneys and in downstream exhaust systems of other processes of combustion of fossil materials.

### Technical Problem

In light of the above discussion, it has become particularly important to optimize processes of $CO_2$ conversion induced by mechanical activation under continuous gas flow conditions, in particular as a gas mixture having a $CO_2$ content (approx. 8-10%) comparable to the above-mentioned data.

Therefore, the need is still felt by those skilled in the art for an economical and environmentally compatible method of converting carbon dioxide ($CO_2$), either as such or mixed with other gases, into high added value chemical compounds under continuous gas flow conditions.

The object of the present invention is to provide an adequate response to the above-highlighted technical problem.

### Description of the Invention

To such end, the present inventors' attention was focused on processes for the production of hydrogen from water and for $CO_2$ conversion induced by mechanical activation in the presence of mineral Olivine and $H_2O$, and particular attention was paid by the same inventors to studying/optimizing the experimental conditions of the process, so as to make them compatible with the implementation conditions of several industrial-scale processes producing large amounts of $CO_2$. In this regard, a number of preliminary laboratory tests were also carried out, which showed the great potential of olivine in the evolutionary process of molecular hydrogen obtained by mechanochemically activated dissociation of water, as well as in the production of fair amounts of methane. Said preliminary tests were conducted using a mechanochemical reactor in "batch" (discontinuous) mode, and made it possible to evaluate, among other things, some aspects related to the structural evolution of the solid phases involved and to the kinetics of the process under specific experimental conditions. [11]

The present invention relates, on the other hand, to a chemical process, conducted by mechanical activation under continuous gas flow conditions, for converting $CO_2$ into a mixture of high added value chemical compounds comprising a mixture of low molecular weight hydrocarbons, mainly methane, ethylene and ethane, along with solid products of mineral carbonation, mainly Mg and Fe carbonates. Therefore, $CO_2$ conversion is effected by means of a continuous mechanochemical process under gas flow, in the absence of any solvents, starting from the following reagents: Olivine powder (solid solution of Mg and Fe silicates), $H_2O$ (whether or not deionized) and $CO_2$ (or a gas flow containing $CO_2$ in quantities (approx. 8-10%) similar to those previously specified herein for fumes released from post-combustion chimneys of an industrial manufacturing process or the like). The solid reagents are inserted into a suitable mechanochemical reactor constructed (by way of non-limiting example, as previously described) starting from a ball mill driven by, for example, an electric motor (other driving means may however be used, as will be specified hereinafter). The solid phases are subjected to mechanical treatment by suitable milling bodies (e.g. balls made of steel or other suitable materials, or other milling means adequate for the type of reactor in use), and are put under conditions of reaction with the fluidic reagents ($CO_2$, $H_2O$, or a flow of humid gases containing $CO_2$), which in turn are transported into and through the reactor under continuous flow conditions. The process is characterized by the absence of any solvents, by the method of activation of the process (using mechanical energy supplied by the milling bodies to the reagents that are trapped during ball-ball or ball-jar collisions), by very high yield values of the $CO_2$ transformation process, and by very fast reaction kinetics, in addition to rather mild process conditions (e.g. temperature and pressure) comparable to those described above with reference to fumes released from post-combustion chimneys of an industrial manufacturing process or a process of combustion of organic materials (e.g. wood, coal, oil, petrol, diesel fuel, gas and/or biomass).

Such characteristics lend environmental sustainability properties to the process of the invention, resulting in great potential interest in the commercial exploitation of said process.

The mechanism through which the series of chemical transformations of the process occur is certainly complex and comprises several stages, including dissociation of the $H_2O$ molecule, dissociation of the $CO_2$ molecule, surface adsorption processes, chemical absorption processes, bulk scattering, structural evolution of the solid phases, synthesis of hydrocarbons, processes of desorption of gaseous phases from the surfaces of the solids.

A detailed evaluation of such aspects goes beyond the application properties related to the scope of the present invention; it is however of interest here to underline that during the initial phases of the process large amounts of $H_2$ are produced, i.e. hydrogen in molecular form (with reference to the reaction under examination), originated from the dissociation of water promoted by the kinetic energy derived from the impact of the milling bodies against the humid Olivine powder. Formation of $H_2$ is also correlated with oxidation processes (Fe2+ to give Fe3+) that can be observed in the solid phases that constitute Olivine.

Once hydrogen has been produced, in the presence of $CO_2$ it shows to be able to induce carbon dioxide reduction, again thanks to the mechanical energy generated and transferred during the non-elastic collisions occurring between the milling bodies, the olivine powder and the reagents in liquid and gaseous phase.

In summary, it is the object of the present invention to provide a continuous mechanochemical process under gas flow to carry out the conversion of $CO_2$ into a mixture of high added value chemical compounds, said mixture comprising a mixture of low molecular weight hydrocarbons, mainly methane, ethylene and ethane, and solid products of mineral carbonation, mainly Mg and Fe carbonates, said process comprising at least the following steps:

a)- continuously passing a gas flow comprising, or consisting of, $CO_2$ through a reactor equipped with milling means, and in the presence of Olivine powder and water, in liquid or gaseous form, at from room temperature to $\leq$ 100 °C; said reactor being subjected to motion in such a way that the milling means trigger a mechanochemical reaction that, on one side, produces hydrogen from the present water, and then said hydrogen carries out the conversion of a portion of $CO_2$ into said low molecular weight hydrocarbons, while, on the other side, the other portion of $CO_2$ reacts with the transformation products of Olivine and non-reacted $H_2O$ to give said products of mineral carbonation;
b)- separating and recovering from the gas mixture exiting the reactor said low molecular weight hydrocarbons and non-reacted hydrogen obtained in step a);
c)- at the end of the reaction, separating and recovering from the reactor said solid products of mineral carbonation of olivine obtained in step a).

The advantages of the technique (process) of the present invention, as described above, include at least:

A) use of a low-cost natural mineral (Olivine: about 13 USD/ton) ;

B) generation of the hydrogen necessary for the process of chemical reduction of carbon dioxide into methane according to the following reaction scheme:

$$CO_2 + 4H_2 \longrightarrow CH_4 + 2H_2O$$

directly from the water contained in the gas mixture;

C) execution of the conversion process at a low temperature (from room temperature to approx. $\leq 100°C$; preferably, to approx. $\leq 80°C$; more preferably, to approx. $\leq 60°C$ or $\leq 40°C$) ;

D) further use of the solid materials thus obtained, which, once exhausted, are inert in terms of toxicity and chemical noxiousness, and any metals that may be present therein can be extracted using methods known in the literature; they are also totally reusable in the cement industry (e.g. to give magnesia cement), thus not representing a problem for the environment;

E) production of energy vectors (hydrogen and methane and light hydrocarbons, e.g. ethane and ethylene) and construction materials (magnesium oxide - magnesia cement).

**Brief Description of the Figures**

**Figure 1** shows an illustrative embodiment of the experimental apparatus of the present invention.

**Figure 2** represents the relationship between conversion (X), selectivity (S) and yield (Y) when products B and C are obtained from the reagents, indicated as A, plus a certain quantity of non-reacted reagent A. At time t1 the process has not yet begun, and only the reagents (A) are present. At time t2, following the start of the process, the system will be composed of the products (B and C) and a part of reagents (A) not yet reacted.

**Figures 3a)** and **3b)** show, respectively, the $CO_2$ conversion rate and the concentration, expressed in ppm, of the products obtained, as recorded during the grinding process.

**Figures 4a)** and **4b)** show, respectively, the selectivity of the products obtained during the grinding process and the estimated yield of the products.

**Figure 5** shows a comparison among tests conducted with, respectively, 0.15, 0.3 and 0.6 ml of $H_2O$ and 2g of Olivine.

**Figure 6** shows the $CO_2$ conversion rate, evaluated by using milling bodies having different masses.

**Figure 7** shows $CO_2$ conversion as a function of grinding time. The two curves refer to tests conducted with different values of the revolution speed of the mill, as indicated in the box.

**Figure 8** represents a comparison that shows $CO_2$ conversion as a function of time with Olivine at its first use (grey curve) and with Olivine at its second use (black curve).

**Figure 9** shows the diffraction patterns of unprocessed Olivine (bottom) and of the post-reaction materials treated under different conditions, as described in this specification.

**Figure 10** shows the diffraction patterns of unprocessed Olivine (bottom) and of a representative sample of processed Olivine (top). At its base, the graph shows the sequences of the characteristic signals of the main crystallographic phases involved.

**Figures 11 A** and **11 B** show, respectively, an SEM micrography of a sample of Olivine pre-ground for 1 hour and an SEM micrography of a sample of Olivine processed in the presence of water and carbon dioxide for a time longer than one hour.

**Figure 12** shows the map of elements and the EDX microanalysis. The upper part shows the map of elements generated on a sample of Olivine processed by mechanochemical activation with water and carbon dioxide. The lower part shows the fluorescence spectrum, with the characteristic signals of the existing elements. The box shows the mean percentages of the detected elements.

**Figure 13** shows the FT-IR spectra of unprocessed and processed (i.e. subjected to mechanochemical reaction) Olivine. Prior to recording the spectra, the samples were dried in a stove at 80°C to remove all traces of humidity.

**Figure 14** shows the evolution of the non-reacted hydrogen produced during the $CO_2$ activation process.

**Experimental Section**

Description of the Reactor and Reaction Conditions

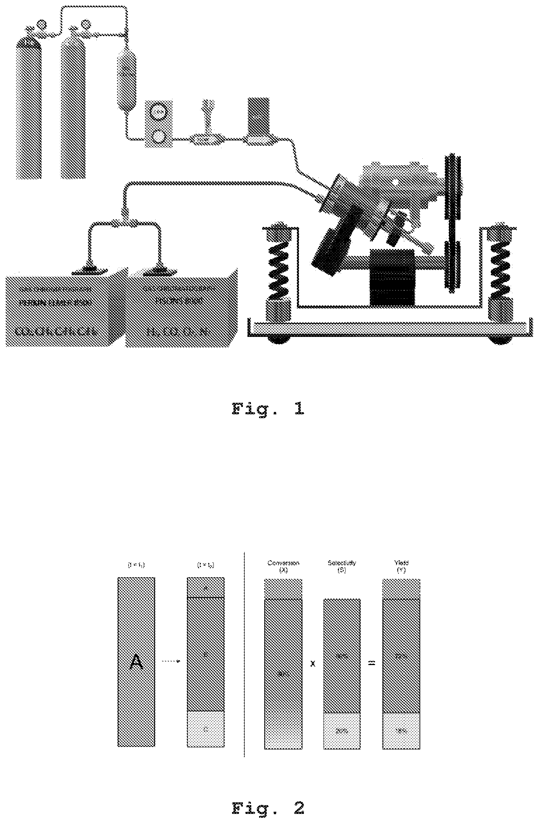

The following experimental section will describe, by way of illustrative example, some of the characteristic aspects of the present invention, without however by no means limiting the broad application potential thereof. Based on the following explanations, those skilled in the art will have no difficulty in applying and modifying the teachings provided herein by adapting the dimensions, quantities and construction aspects to specific practical implementations. In one embodiment, the equipment used for the experimental activity is schematized in Figure 1. It essentially consists of a cylindrical reactor (a grinding jar (mill) having an internal volume of approx. 65 ml), suitable for the execution of mechanical treatments by means of ball milling processes. The reactor and the milling bodies are made of hardened stainless steel (of course, the dimensions and materials of the reactor may be freely selected and/or defined for specific applications, depending on the type and rate of gas flow (e.g. an exhaust gas) to be treated. The same also applies to the number, size and material of the milling means). The cylindrical reactor is equipped with gas-tight covers and gas transfer lines. The gas lines, e.g. partly made of steel and partly consisting of flexible Teflon (PTFE) hoses, provide the connection between the reagent gas supply (or the exhaust gas flow) and the reactor, and between the latter and the two gas chromatographs for analyzing the reagent and the produced gases. Such a "flow" configuration of the reactor allows the execution of tests under a continuous supply of gaseous reagents. The gas chromatographs are both equipped with detectors suitable for a quantitative evaluation of the gaseous phases of interest. A first gas chromatograph, equipped with a GS-Q wide-bore capillary column and TCD-FID detectors, with He as a carrier gas, made it possible to evaluate the $CO_2$ through the TCD detector and the low molecular weight hydrocarbons through the FID detector; a second gas chromatograph, equipped with a column packed with 13X (10Å) molecular sieves and a TCD detector, with Ar as a carrier gas, made it possible to evaluate the amounts of $H_2$, CO, $O_2$ and $N_2$.

The mechanochemical reactor was housed in the seat of a known Spex Mixer/Mill mod.8000, [Spex Certiprep., https://www.spexcertiprep.com], suitably modified and equipped with a three-phase electric motor controlled by a speed regulator, which provides control over the number of revolutions of the motor shaft and imparts to the mill a three-dimensional motion in space. The tests were conducted under two different conditions: at 875 and 1,000 revolutions per minute (rpm) of the motor shaft, respectively corresponding to 14.5 and 16.6 Hz.

In practice, the revolution speed of the reactor can be varied, depending on the type of application, from 500 rpm to 1,500 rpm; preferably, from 600 rpm to 1,400 rpm; more preferably, from 700 rpm to 1,300 rpm; even more preferably, from 800 rpm to 1,200 rpm.

The tests were conducted using solid samples having a mass of 2g of commercial Olivine (produced by SATEF-HA, Italy). The latter was subjected to a mechanical pre-treatment in Air atmosphere for 1h using 3 balls (made of stainless steel) having a mass of 4 g each, for the purpose of homogenizing the powder size. At the end of the pre-treatment, 0.3 ml of deionized $H_2O$ were added to the Olivine powder.

The above-described conditions correspond to an Olivine/$H_2O$ stoichiometric ratio of 1:2.

The reactor was connected to the gas lines and housed in the support of the Spex 8000 commercial mill, for the execution of the reactive tests.

The gaseous reaction mixture was composed of $CO_2$ (Sapio, purity of 99.995), with abundance of 10% by volume in He (Sapio, BIP purity). After saturating the atmosphere of the mechanochemical reactor, the reactive mixture was fed in a flow of 10 ml/min, kept constant through a regulation valve and measured with a mass flowmeter (in the applications of the process of the invention, the flow rate may be changed freely according to the amount of exhaust fumes containing $CO_2$ released per time unit, the chimney size, and other parameters related to the release of said fumes). The composition of the reactive mixture for the tests was selected in tight relation with the average composition data of the mixture containing $CO_2$ exiting the above-mentioned industrial combustion processes. In these tests, the matrix was He, instead of $N_2$, in order to avoid any side effects due to a possible interaction between $N_2$ and Olivine and to avoid any overlapping effects in the analyses of the permanent gases. This will not limit the applicability of the invention, which may also be implemented by the operator with $N_2$ as a gaseous matrix.

Description of the Results of the $CO_2$ Conversion Tests and of the Analyses of the Solid and Gaseous Phases

The following will present the results of the $CO_2$ conversion tests under the above-described conditions. Data will be provided about the analyses of the gaseous phases conducted by gas chromatography, the analyses of the solid phases conducted by X-ray diffraction techniques, and the morphological and elementary analyses conducted on the solid phases by, respectively, scanning electron microscopy (SEM) and X fluorescence.

The mechanical treatment in reactive atmosphere was conducted by using 3 steel balls having a mass of 4 g each and by setting the revolution speed of the motor to 875 rpm, unless otherwise specified. The kinetics of the $CO_2$ conversion process was monitored by evaluating the degree of $CO_2$ conversion via measurement of the concentration of $CO_2$ eluted by the reactor after selected mechanical treatment times up to 180 minutes.

Definition of the Descriptor Parameters of the Process

The study of the process was conducted by monitoring, as parameters, the rate of $CO_2$ "*conversion*", the "*selectivity*" in giving the products, and the "yield" of a specific product. Figure 2 illustrates, in graphic form, the meaning of these three descriptor parameters.

*CO2 percent conversion*

It indicates the rate of $CO_2$ transformation compared to the initial concentration, and is expressed by the following mathematical relation:

$$\textbf{CO}_2 \textbf{ conversion, \%} = \frac{[CO_2]_i - [CO_2]_t}{[CO_2]_i} \cdot \textbf{100}$$

where " $[CO_2]_i$" indicates the $CO_2$ concentration at time "i=initial", i.e. before the start of the process;
where " $[CO_2]_t$" indicates the $CO_2$ concentration at time "t", i.e. at a time instant after the start of the process.

In gas chromatography, the concentration of an analyte can be expressed as the product of a response factor and the area of the signal of the analyte under evaluation. From a mathematical viewpoint, this can be expressed as:

$$[\textbf{CO}_2] = \textbf{f}_r \cdot \textbf{Area of CO}_2 \textbf{ signal}$$

where "$[CO_2]$" is the concentration of the carbon dioxide analyte; and
where "$f_r$" is the response factor for the $CO_2$ analyte and refers to a specific column and a specific method of analysis.

Therefore, with appropriate substitutions and simplifications, the equation for calculating the $CO_2$ conversion rate can be rewritten as:

$$CO2 \text{ conversion, } \% = \frac{(f_r \cdot Area\ CO_{2_i}) - (f_r \cdot Area\ CO_{2_t})}{(f_r \cdot Area\ CO_{2_i})} \cdot 100$$

$$CO2 \text{ conversion, } \% = \frac{f_r \cdot (Area\ CO_{2_i} - Area\ CO_{2_t})}{f_r \cdot Area\ CO_{2_i}} \cdot 100$$

$$\textbf{CO}_2 \text{ conversion, } \% = \frac{Area\ CO_{2_i} - Area\ CO_{2_t}}{Area\ CO_{2_i}} \cdot \textbf{100}$$

*Selectivity*

It expresses the ratio between the concentration of a specific product and the total product concentration. It can be calculated by means of the following mathematical relation (expressed for methane):

$$CH_4 \text{ Selectivity, } \% = \frac{[CH_4]}{[X]} \cdot 100 \ ; \qquad [X] = \text{total product concentration}$$

This relation is applied in order to obtain the selectivity of the detectable products, taking into account that, for each one of them, it will be necessary to draw a calibration straight line in order to be able to obtain the concentration.

*Yield of the reaction in giving methane (and/or other products)*

It expresses the percent abundance of a product relative to the conversion rate of the process. From a mathematical viewpoint, it can be expressed as the product of selectivity and conversion:

$$CH_4 \text{ Yield, } \% = CH_4 \text{ Selectivity} \cdot CO_2 \text{ Conversion}/100$$

Results

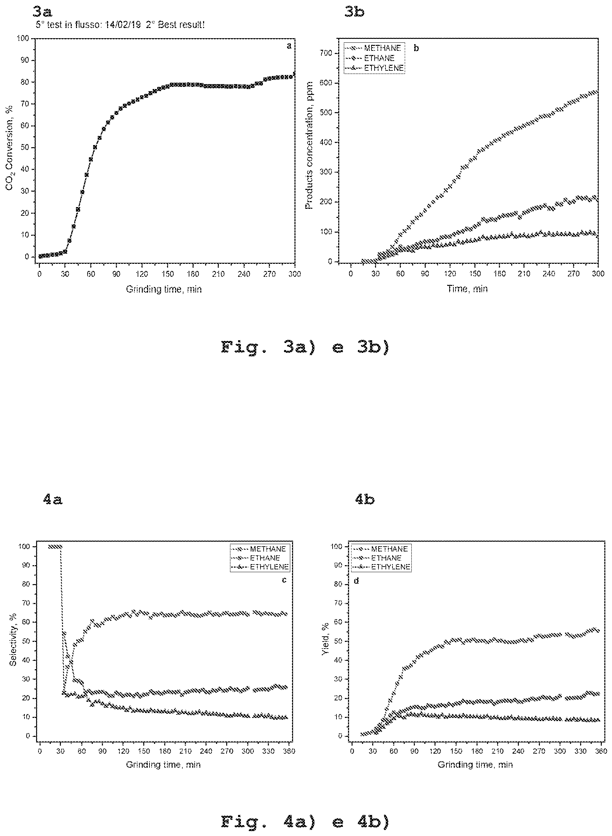

The test results, expressed as percent values of $CO_2$ conversion over time (Figure 3a), highlighted very good reproducibility characteristics, with a variability percentage of less than 5% (attributable to the experimental/instrumental error), and Figure 3a) shows a typical profile of the experimental data of the reaction kinetics. The pattern shows a sigmoidal $CO_2$ conversion profile. In all tests carried out, conversion grows significantly only after an initial induction time, which in the test shown in Figure 3a) was approximately 30 minutes. Conversion speed then increases, reaches a maximum value at an inflection point in the curve, and then decreases, corresponding to the asymptote the conversion tends to, which in the case represented in Figure 3a) exceeds the value of 80%. As can be seen in Figure 3a), 150 minutes after the start of the grinding process the $CO_2$ conversion rate remained almost constant for 3 hours. The sigmoidal pattern of the kinetic curve is typical of many mechanochemical processes, and particularly of processes involving solid-gas heterogeneous systems. Such a pattern has often been interpreted as the result of *nucleation-and-growth* processes and analyzed on the basis of Avrami-Erofeev mechanisms. [11] Without going into details of the mechanistic analysis, it is however useful to underline, in this context, that the shape of the kinetic curve profile comprises multiple elementary stages of the conversion process, including superficial $H_2O$ adsorption by Olivine, its dissociation, processes of $H_2$ and $O_2$ scattering in the bulk of the solid phase, oxidative processes undergone by $Fe2+$, adsorption and absorption of $CO_2$, its reduction by $H_2$, mineral carbonation processes, scattering and desorption of the formed hydrocarbons, etc. The exact correlation between such processes and the shape of the curve goes beyond the intention of the present invention, but they should be taken into consideration for a global evaluation of the process. A further aspect is the analysis of the hydrocarbon phases produced: the process highlights how a $CO_2$ fraction undergoes a reduction process and hence can be transformed into light hydrocarbons such as methane, ethylene and ethane. The kinetic curves concerning the formation of such gaseous phases are shown in Figure 3b): the profile of such curves seems to follow the same sigmoidal pattern as the one previously mentioned, and the concentration values of said phases are different. The maximum concentrations measured for the three above-mentioned products are 680 ppm for methane, 280 ppm for ethane and 100 ppm for ethylene, measured after 330 minutes of grinding time (Figure 3b)). Figure 4a) shows the trend over time of the selectivity data for the above-mentioned products: methane is the main product, with a selectivity of 60%, while the selectivity values for ethane and ethylene are, respectively, 20-25% and 8-10%.

Figure 4b shows global yield data, indicating a production of approximately 50% for methane, 10-15% for ethane and 10% for ethylene.

It must be pointed that the conversion, selectivity and yield data are very stable over time, with values reaching a maximum point after approximately 90 minutes and then remaining constant (except for the experimental error) throughout the observation interval, i.e. for up to 360 minutes of mechanical treatment.

In order to deepen the knowledge of the process mechanism and determine the experimental conditions that led to the best results in terms of conversion, selectivity and yield, the process was studied as a function of some experimental parameters, in particular the stoichiometric ratio between $H_2O$ and Olivine and the mechanical energy transferred to the reagents during the mechanical treatment.

Dependency of the Kinetics on the Olivine/$H_2O$ stoichiometric ratio

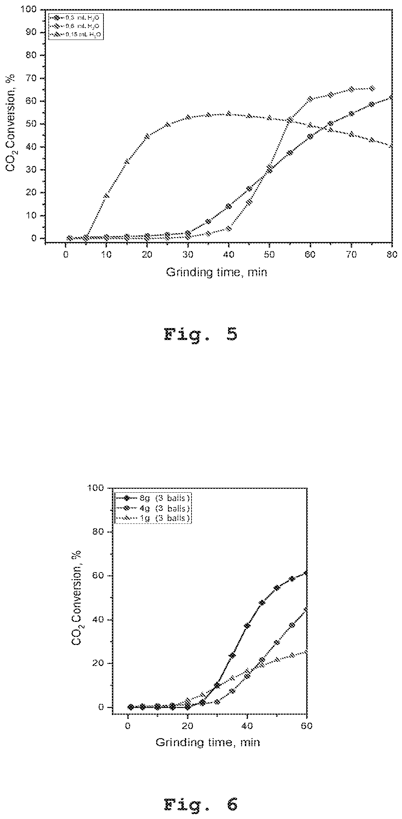

The $H_2O$ content in the reaction environment represents an important parameter in the reaction of methanation of $CO_2$ mechanically activated in the presence of Olivine, because water is the hydrogen source. Figure 5 shows the results of tests conducted with different $H_2O$ contents in the reaction environment.

The corresponding stoichiometric ratio values are as follows:

0.15 ml $H_2O$, Olivine/$H_2O$ = 0.01 mol Olivine/0.01 mol $H_2O$ = 1:1 *
0.3 ml $H_2O$, Olivine/ $H_2O$ = 0.01 mol Olivine/0.02 mol $H_2O$ = 1:2 *
0.6 ml $H_2O$, Olivine/ $H_2O$ = 0.01 mol Olivine/0.03 mol $H_2O$ = 1:3 *
*Such stoichiometric ratios were calculated considering Olivine powders as consisting only of Forsterite Ferroan phase of formula ($Fe_{0,184}$, $Mg_{1,816}$)$SiO_4$ and molar mass of 146.4693 g/mol.

The kinetic curves shown in Figure 5 highlight the existence of an initial period during which $CO_2$ conversion appears to be negligible. Such induction period seems to be influenced by the adopted experimental conditions, and its amplitude depends on the relative ratio between Olivine and $H_2O$. In the three tests presented herein, therefore, the conversion data increase according to a sigmoidal kinetic profile, whose specific characteristics seem to differ (maximum conversion speed values and mechanical treatment time values at which measurements were taken). The maximum conversion value obtained during the test appears to be similar (approx. 60%) in all three cases. It should be noted that, in the test conducted with the lowest (1:1) Olivine/$H_2O$ ratio, the rate of conversion decreases in a monotonous manner after having reached the maximum value. This seems to suggest that, under these conditions, the $H_2O$ content of the

reaction cannot keep constant the $CO_2$ conversion value, although at this level it is not possible to discern whether the limiting factor is Fe2+ oxidation or CO2 reduction.

Dependency of the Kinetics on the Energy Transfer Conditions During the Mechanical Treatment

The present study is based on the activation of the process of conversion of carbon dioxide induced mechanochemically. With such mode of activation, the energy required for triggering the chemical process is administered in the form of kinetic energy ($E_k$=1/2 mv$^2$): the dependency of the reaction kinetics on the mechanical energy transferred to the reagents was evaluated by means of tests conducted under different conditions in terms of the mass of the milling bodies and the revolution speed of the motor driving the Spex mill.

As far as the former parameter is concerned, i.e. the mass of the milling bodies, several tests were carried out using three steel balls having a mass of, respectively, 1g each, 4g each, and 8g each, as highlighted by the three curves shown in Figure 6.

Figure 6 shows, again, sigmoidal patterns for each one of the three curves referring to different experimental conditions as concerns the masses of the milling bodies employed in the tests. The time of mechanical treatment being equal, the degree of $CO_2$ conversion depends on the mass of the milling bodies in use, and conversion speed is also dependent on the same parameter. In general terms, it is important to point out that it is possible to determine the kinetic energy transferred during each collision, but such evaluation lies outside the present context, while still being important in view of an in-depth mechanistic survey.

Conversely, variations in the mass of the milling bodies do not seem to significantly affect the selectivity values for gaseous products like methane, ethylene and ethane, obtained during the process. In qualitative terms, these patterns follow those shown in Figure 4a).

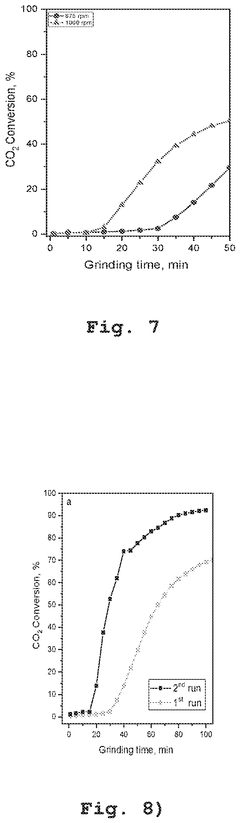

The energy transferred to the reagent species through collisions with the milling bodies can also be modulated by adjusting the revolution speed of the electric motor connected to the Spex 8000 mill. The rotation of the motor shaft of the mill imparts motion to the milling bodies and affects the frequency of the collisions between the balls and the jar walls. The tests were carried out using commercial Spex Mixer/Mill 8000 mills at revolution speeds of 875 rpm and 1,000 rpm. The results concerning the dependency of the $CO_2$ conversion data on the revolution speed of the motor of the mill are shown in Figure 7.

The revolution frequency of the motor of the mill affects induction time: with a revolution speed of 1,000 rpm, the $CO_2$ conversion process is activated sooner than with a revolution speed of 875 rpm. In this case as well, the distribution of the final products obtained follows the pattern shown in Figure 4a).

The study also tackled the behaviour of the systems used for the mechanically activated $CO_2$ conversion process when re-processed in the presence of $CO_2$ and $H_2O$. Figure 8 shows a comparison between the experimental data curves obtained with Olivine powders at their first use (grey curve) and at their second use (black curve).

It emerges that the system is more active at the second use: induction time is shorter, and the $CO_2$ conversion value reaches 95%, i.e. it is higher than obtained in all tests carried out with Olivine at the "first use".

This fact confirms the thesis that the proposed material (Olivine) is a very good candidate for use in the process of transforming $CO_2$ into products of high commercial value. The distribution of the products thus obtained follows the same pattern as shown in Figure 4a).

Morphological-Structural Characterization of the Solid Materials Before and After the Mechanochemical Reaction

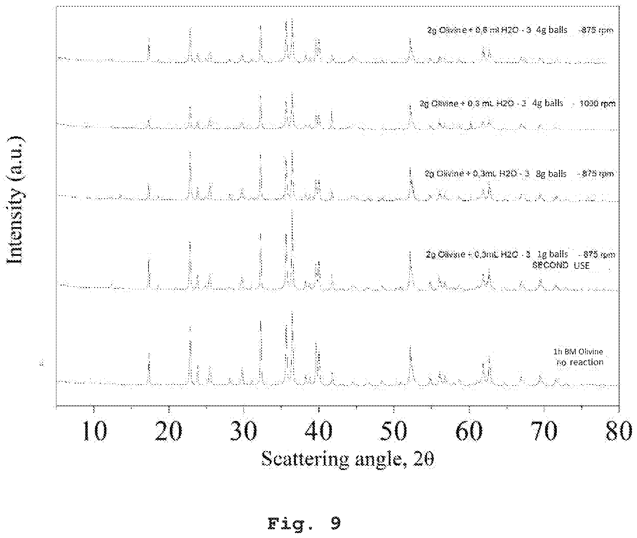

Olivine powders before (pre-grounded for 1 hour) and after the reaction were characterized by X-ray diffraction using a Rigaku SmartLab diffractometer with a rotating anode source ($\lambda_{Cu}$ = 1.54178 Å) and equipped with a graphite monochromator on the diffracted beam, employing a Bragg-Brentano geometry. Figure 9 shows a sequence of X diffraction patterns concerning unprocessed Olivine and samples obtained after several mechanochemical tests under the conditions specified in Figure 9. From the sequence of diffraction patterns for the analyzed systems summarized in Figure 9, one can see that in the processed materials the main phases of unprocessed Olivine (Forsterite, Enstatite e Clinochlore) are preserved, with the appearance of carbonate phases such as Nesquehonite (magnesium carbonate hydrate) and Iron. Other minority phases (< 1% by mass) not yet attributed, but compatible with phases such as Magnesite (magnesium carbonate), Maghemite (Fe(III) oxide) were also detected.

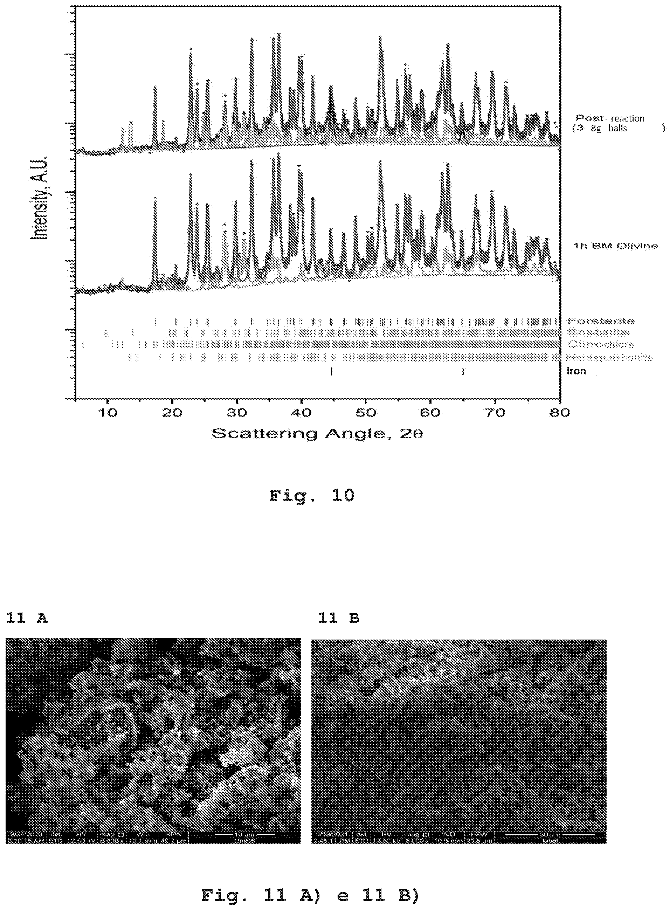

The quantitative evaluation of the abundance of the crystallographic phases in the different samples, and of the microstructural parameters, was conducted using the Rietveld refinement method on all samples under examination. Figure 10 shows, by way of example, the X diffraction patterns concerning two samples representative of the tests carried out: Olivine after pre-treatment (lower pattern) and Olivine after mechanochemical treatment with $CO_2$ and $H_2O$ (upper pattern in Figure 10). Along with the experimental data, the figure shows the profiles concerning the structural refinement made in accordance with the Rietveld method, and the following Table 1 and Table 2 show the results of the analysis of the abundance of the crystallographic phases and of the microstructural parameters.

The X-ray diffraction pattern of unprocessed Olivine can be described through the Forsterite (90% by mass), Enstatite (8%) and Clinochlore (2%) phases, whereas the samples processed in the reactive atmosphere show the Forsterite (70-86%), Enstatite (5-7%), Clinochlore (6-15%), Nesquehonite (1-3.6%) phases, and a minority fraction of elementary Iron (1-3%) can be recognized (the latter may be due to a phenomenon of precipitation of Fe particles from the walls of the mechanochemical reactor during the process).

Table 1: Crystallographic data concerning the phases in the unprocessed Olivine sample, subjected to pre-grinding for 1 hour. The quantitative evaluation was obtained by analyzing the X-ray diffraction data with the Rietveld method.

| Name | Chemical Formula | AMCSD code | a/Å | b/Å | c/Å | Grain sizes nm | Micro Strain |
|---|---|---|---|---|---|---|---|
| Forsterite | (Fe0.184, Mg1.816) SiO4 | 1696 | 4,163 | 10,225 | 5,994 | 103,25 | 2,33E-06 |
| Enstatite | (Fe0.155, Mg0.845) SiO3 | 1695 | 18,253 | 8,833 | 5,200 | 41,15 | 2,11E-04 |
| Clinochlore | [(Al1.84, Fe0.5, Mg4.5) Si3.16O18H8] | 4250 | 5,162 | 9,562 | 14,418 | 153,25 | 3,74E-03 |

Table 2: Crystallographic data concerning the phases contained in the Olivine sample after mechanochemical treatment with $CO_2$ and $H_2O$. The quantitative evaluation was obtained by analyzing the X-ray diffraction data with the Rietveld method.

| Name | Chemical Formula | AMCSD code | a/Å | b/Å | c/Å | Grain sizes nm | Micro Strain |
|---|---|---|---|---|---|---|---|
| Forsterite | (Fe0.184, Mg1.816) SiO4 | 1696 | 4,762 | 10,223 | 5,993 | 106,59 | 4,40E-06 |
| Enstatite | (Fe0.155, Mg0.845) SiO3 | 1695 | 18,255 | 8,833 | 5,196 | 46,83 | 1,81E-04 |
| Clinochlore | [(Al1.84, Fe0.5, Mg4.5)Si3.16O18H8] | 4250 | 5,193 | 9,552 | 14,398 | 156,78 | 1,46E-03 |
| Nesquehonite | MgCO3·3(H2O) | 9432 | 7,767 | 5,375 | 1,213 | 119,23 | 1,93E-05 |
| $\alpha$-Fe | Fe | 670 | 2,872 | 2,872 | 2,872 | 32 | 2,18E-03 |

The unprocessed materials and the processed materials (i.e. those subjected to mechanochemical reaction) were analyzed by scanning electron microscopy (SEM) using an FEI Quanta 200 microscope equipped with a Genesis XM21 Apollo 10SSD detector for EDS microanalyses.

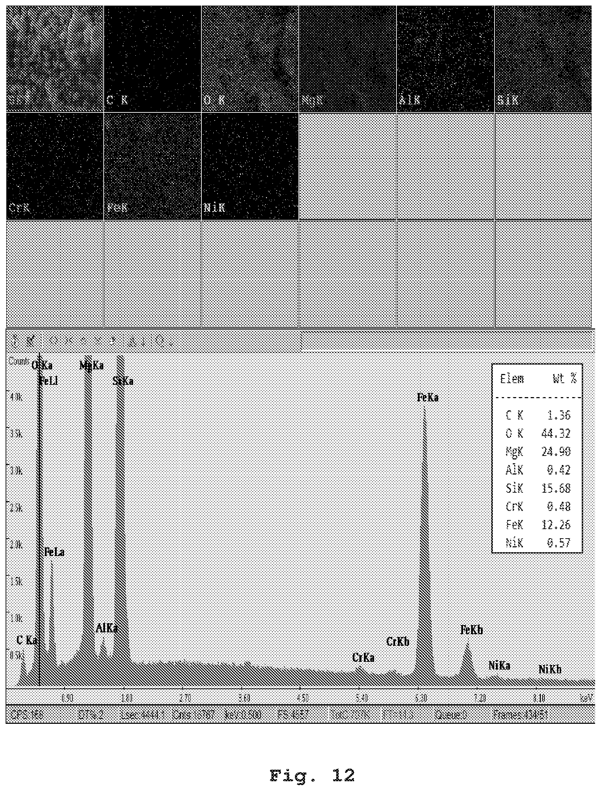

Figure 11 shows the images obtained by means of the ETD detector, concerning the secondary electrons emitted by the samples, and illustrates the morphology of 2 samples of Olivine, respectively pre-ground for 1 hour (Figure 11 A) and processed in the presence of $CO_2$ and $H_2O$ (Figure 11 B). The samples show no substantial differences, except for a smaller grain size due to prolonged exposure to grinding. In the map of the distribution of elements in the processed Olivine samples, it is possible to observe the presence of Fe, Mg, Al, Cr, Si, O, that constitute the silicate, and carbon C, the presence of which is due to the formation of carbonates following the process of mechanochemical activation in the presence of $CO_2$ (Figure 12).

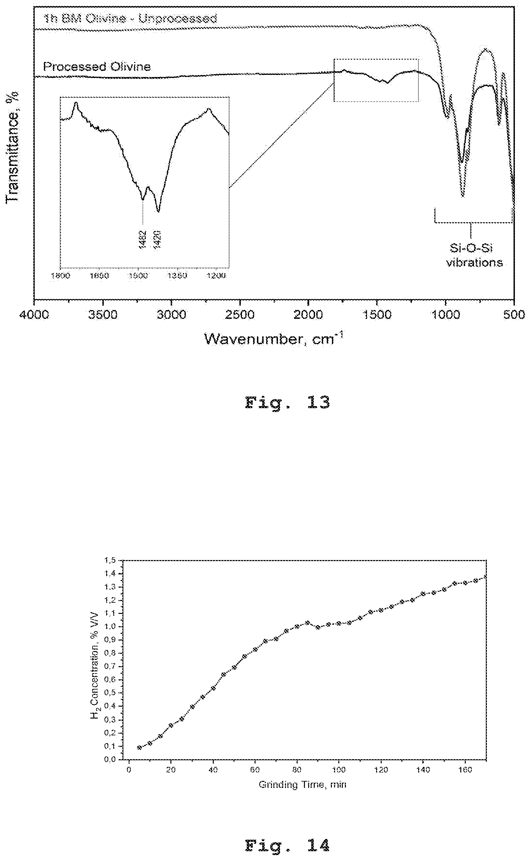

Pre-reaction and post-reaction samples were also characterized by infrared spectroscopy (FT-IR) using a Jasco FT-IR 4600 instrument in ATR mode. Figure 13 shows the FT-IR spectra of unprocessed Olivine and Olivine subjected to the mechanochemical reaction.

The signals between 1,000 and 500 cm⁻¹ can be attributed to stretching and bending vibrations of the Si-O-Si bonds that are characteristic of silicates; such bands are easily identifiable in both spectra. In the spectrum concerning the sample of mechanically processed Olivine, it is also possible to observe the presence of two new characteristic bands centered at 1,482 and 1,420 cm⁻¹, which are compatible with the vibrations of the carbonate groups, whose appearance is due to the presence of $CO_2$ during the grinding process.[12][13] Such signals confirm the evidence obtained from the X-ray diffraction data.

Hydrogen Evolution

An experiment was also conducted in order to evaluate the process of evolution of hydrogen, which did not participate in the reaction with $CO_2$, originated from the dissociation of water induced by the mechanochemical process in the presence of Olivine. This test was carried out under flow conditions, using a mixture of 10% $CO_2$ balanced in Argon. The experimental setup can be summarized as follows:

- 2g of Olivine pre-ground for one hour;
- $H_2O$: 0.3 mL;
- Milling bodies: 3 balls weighing 4 g each;
- Revolution speed of the mill motor: 875 rpm.

The test was conducted in accordance with the same operating modes as described in the section entitled "Description of the Invention". The experimental data concerning hydrogen evolution are shown in Figure 14.

Hydrogen is produced starting from water by means of a dissociation process activated by the mechanochemical process in the presence of Olivine. By appropriately dosing the amount of $H_2O$ and the Olivine/$H_2O$ ratio in the reactor, it is possible to keep such production constant (which is a fundamental requisite) during the methanation process.

**Industrial Applicability of the Invention**

The method of the present invention has made it possible to convert carbon dioxide ($CO_2$) into high added value chemical compounds under continuous gas flow conditions. In particular, said method converts $CO_2$ into a mixture of high added value chemical compounds comprising low molecular weight hydrocarbons, mainly methane, ethylene and ethane, and products of mineral carbonation, mainly Mg and Fe carbonates. Said $CO_2$ conversion is achieved through a mechanochemical process and is advantageously applicable to any activity emitting into the atmosphere exhaust fumes comprising, among other possible gaseous substances, significant amounts of $CO_2$.

**List of References**

[1] https://www.climate.gov/news-features/understanding-climate/climate-change-atmospheric-carbon-dioxide
[2] J. Wanga et al., International Ocean and Polar Engineering Conference, Sapporo, Japan, June 10-15 (2018)
[3] https://climate.nasa.gov/effects/
[4] M. Rosa et al., Journal of CO2 Utilization, 9 (2015) 82-102
[5] A.S. Agarwal et al., ChemSusChem 4 (2011) 1301-1310
[6] Hydrogen in a low-carbon economy, Committee on Climate Change, London, November 2018
[7] A. Álvarez et al., Chem. Phys. Chem., 18 (2017) 3135 - 3141
[8] C. Oze et al., Geophysical Research Letters - 32 (2015) L10203
[9] F. Wang et al., Minerals Engineering - 131 (2019) 185-19
[10] M. McCollom et al, Geochem.Cosmochim. Acta 65 (2001) 3769-3778
[11] V. Farina et al., Front. Energy Res., 7 (2019) 107
[12] E. Turianicová et al, «A POSSIBLE WAY TO STORAGE CARBON DIOXIDE ON MECHANICALLY ACTIVATED OLIVINE (Mg,Fe)2SiO4,» INCOME 2008, Jamshedpur, India, 2008
[13] F. Torre et al, Powder Technology, 364 (2020) 915-923

**Claims**

1. A continuous mechanochemical process under gas flow to carry out the conversion of $CO_2$ into a mixture of high added value chemical compounds, said mixture comprising a mixture of low molecular weight hydrocarbons, mainly methane, ethylene and ethane, and solid products of mineral carbonation, mainly Mg and Fe carbonates, said process comprising at least the following steps:

   a)- continuously passing a gas flow comprising, or consisting of, $CO_2$ through a reactor equipped with milling means, and in the presence of Olivine powder and water, in liquid or gaseous form, at from room temperature to $\leq 100°C$; said reactor being subjected to motion in such a way that the milling means trigger a mechanochemical reaction that, on one side, produces hydrogen from the present water, and then said hydrogen carries out the conversion of a portion of $CO_2$ into said low molecular weight hydrocarbons, while, on the other side, the other portion of $CO_2$ reacts with the transformation products of Olivine and non-reacted $H_2O$ to give said products of mineral carbonation;

b)- separating and recovering from the gas mixture exiting the reactor said low molecular weight hydrocarbons and non-reacted hydrogen obtained in step a);

c)- at the end of the reaction, separating and recovering from the reactor said solid products of mineral carbonation of Olivine obtained in step a).

2. The process according to claim 1, wherein said reactor is a mill, or a jar, equipped with milling means consisting of rotating spherical bodies (balls); said mill and said balls being made of an abrasion resistant material, hardened stainless steel.

3. The process according to claim 1 or 2, wherein the motion to which the reactor is subjected is a rotary motion at a speed of 500 rpm to 1,500 rpm.

4. The process according to claim 3, wherein said rotary motion occurs at a speed of 600 rpm to 1,400 rpm.

5. The process according to any one of the preceding claims, wherein the stoichiometric ratio of Olivine to $H_2O$ is in the range of 1:1 to 1:3.

6. The process according to claim 5, wherein the stoichiometric ratio of Olivine to $H_2O$ is 1:2.

7. The process according to any one of the preceding claims, wherein the highest measured concentrations of the low molecular weight hydrocarbons produced in step a) are 680 ppm for methane, 280 ppm for ethane, and 100 ppm for ethylene.

8. The process according to claim 7, wherein methane is the main product with a selectivity of 60%, while the selectivity values for ethane and ethylene are 20-25% and 8-10%, respectively.

9. The process according to claim 7 or 8, wherein the global yield in methane production is 50%, while it is 10-15% for ethane and about 10% for ethylene.

10. Use of the process according to any one of the preceding claims to carry out the conversion of $CO_2$ into a mixture of high added value chemical compounds; said mixture comprising a mixture of low molecular weight hydrocarbons, mainly methane, ethylene and ethane, and solid products of mineral carbonation, mainly Mg and Fe carbonates.

**Patentansprüche**

1. Kontinuierliches mechanochemisches Verfahren unter Gasfluss zur Umwandlung von $CO_2$ in ein Gemisch von chemischen Verbindungen mit hohem Mehrwert, wobei das Gemisch ein Gemisch von Kohlenwasserstoffen mit niedrigem Molekulargewicht, hauptsächlich Methan, Ethylen und Ethan, und feste Produkte der Mineralkarbonisierung, hauptsächlich Mg- und Fe-Karbonate, umfasst, wobei das Verfahren mindestens die folgenden Schritte umfasst

a)- kontinuierliches Durchleiten eines Gasstroms, der $CO_2$ enthält oder daraus besteht, durch einen mit Mahlmitteln ausgestatteten Reaktor in Gegenwart von Olivinpulver und Wasser in flüssiger oder gasförmiger Form bei einer Temperatur von Raumtemperatur bis $\leq 100°C$; wobei der Reaktor so in Bewegung gesetzt wird, dass die Mahlmittel eine mechanisch-chemische Reaktion auslösen, die auf der einen Seite aus dem vorhandenen Wasser Wasserstoff erzeugt, der dann die Umwandlung eines Teils des $CO_2$ in die Kohlenwasserstoffe mit niedrigem Molekulargewicht durchführt, während auf der anderen Seite der andere Teil des $CO_2$ mit den Umwandlungsprodukten des Olivins und dem nicht umgesetzten $H_2O$ reagiert, um die Produkte der mineralischen Karbonisierung zu erhalten;

b)- Abtrennung und Rückgewinnung der in Schritt a) erhaltenen niedermolekularen Kohlenwasserstoffe und des nicht umgesetzten Wasserstoffs aus dem Gasgemisch, das den Reaktor verlässt;

c) - am Ende der Reaktion Abtrennung und Rückgewinnung der in Schritt a) erhaltenen festen Produkte der Mineralkarbonisierung von Olivin aus dem Reaktor.

2. Verfahren nach Anspruch 1, bei dem der Reaktor eine Mühle oder ein Gefäß ist, das mit einer Mahlvorrichtung ausgestattet ist, die aus rotierenden kugelförmigen Körpern (Kugeln) besteht, wobei die Mühle und die Kugeln aus einem abriebfesten Material, gehärtetem Edelstahl, hergestellt sind.

3. Verfahren nach Anspruch 1 oder 2, wobei die Bewegung, der der Reaktor unterworfen wird, eine Drehbewegung mit einer Geschwindigkeit von 500 U/min bis 1.500 U/min ist.

4. Verfahren nach Anspruch 3, wobei die Drehbewegung mit einer Geschwindigkeit von 600 U/min bis 1.400 U/min erfolgt.

5. Verfahren nach einem der vorhergehenden Ansprüche, wobei das stöchiometrische Verhältnis von Olivin zu $H_2O$ im Bereich von 1:1 bis 1:3 liegt.

6. Verfahren nach Anspruch 5, wobei das stöchiometrische Verhältnis von Olivin zu $H_2O$ 1:2 beträgt.

7. Verfahren nach einem der vorhergehenden Ansprüche, wobei die höchsten gemessenen Konzentrationen der in Schritt a) hergestellten niedermolekularen Kohlenwasserstoffe 680 ppm für Methan, 280 ppm für Ethan und 100 ppm für Ethylen betragen.

8. Verfahren nach Anspruch 7, wobei Methan das Hauptprodukt mit einer Selektivität von 60 % ist, während die Selektivitätswerte für Ethan und Ethylen 20-25 % bzw. 8-10 % betragen.

9. Verfahren nach Anspruch 7 oder 8, wobei die Gesamtausbeute bei der Methanproduktion 50 % beträgt, während sie bei Ethan 10-15 % und bei Ethylen etwa 10 % beträgt.

10. Verwendung des Verfahrens nach einem der vorhergehenden Ansprüche zur Umwandlung von $CO_2$ in ein Gemisch von chemischen Verbindungen mit hohem Mehrwert, wobei das Gemisch ein Gemisch von Kohlenwasserstoffen mit niedrigem Molekulargewicht, hauptsächlich Methan, Ethylen und Ethan, und feste Produkte der Mineralkarbonisierung, hauptsächlich Mg- und Fe-Karbonate, umfasst.

## Revendications

1. Procédé mécano-chimique continu sous flux gazeux pour réaliser la conversion du $CO_2$ en un mélange de composés chimiques à haute valeur ajoutée, ledit mélange comprenant un mélange d'hydrocarbures de faible poids moléculaire, principalement du méthane, de l'éthylène et de l'éthane, et des produits solides de carbonatation minérale, principalement des carbonates de Mg et de Fe, ledit procédé comprenant au moins les étapes suivantes :

a)- faire passer en continu un flux gazeux comprenant, ou constitué de $CO_2$ à travers un réacteur équipé de moyens de broyage, et en présence de poudre d'Olivine et d'eau, sous forme liquide ou gazeuse, à une température comprise entre la température ambiante et $\leq 100°C$ ; ledit réacteur étant soumis à un mouvement tel que les moyens de broyage déclenchent une réaction mécano-chimique qui, d'une part, produit de l'hydrogène à partir de l'eau présente, puis ledit hydrogène effectue la conversion d'une partie du $CO_2$ en lesdits hydrocarbures de faible poids moléculaire, tandis que, d'autre part, l'autre partie du $CO_2$ réagit avec les produits de transformation de l'olivine et de l'$H_2O$ non réagi pour donner lesdits produits de carbonatation minérale ;
b)- séparer et récupérer dans le mélange gazeux sortant du réacteur les hydrocarbures de faible poids moléculaire et l'hydrogène n'ayant pas réagi obtenus à l'étape a) ;
c)- à la fin de la réaction, séparer et récupérer du réacteur lesdits produits solides de carbonatation minérale de l'olivine obtenus à l'étape a).

2. Procédé selon la revendication 1, dans lequel ledit réacteur est un broyeur, ou une jarre, équipé de moyens de broyage consistant en des corps sphériques rotatifs (billes) ; ledit broyeur et lesdites billes étant fabriqués dans un matériau résistant à l'abrasion, l'acier inoxydable trempé.

3. Procédé selon la revendication 1 ou 2, dans lequel le mouvement auquel le réacteur est soumis est un mouvement rotatif à une vitesse de 500 tours par minute à 1 500 tours par minute.

4. Procédé selon la revendication 3, dans lequel le mouvement rotatif se produit à une vitesse comprise entre 600 et 1 400 tours/minute.

**5.** Procédé selon l'une quelconque des revendications précédentes, dans lequel le rapport stœchiométrique entre l'olivine et $H_2O$ est compris entre 1:1 et 1:3.

**6.** Procédé selon la revendication 5, dans lequel le rapport stœchiométrique entre l'olivine et $H_2O$ est de 1:2.

**7.** Procédé selon l'une quelconque des revendications précédentes, dans lequel les concentrations mesurées les plus élevées des hydrocarbures de faible poids moléculaire produits à l'étape a) sont de 680 ppm pour le méthane, 280 ppm pour l'éthane et 100 ppm pour l'éthylène.

**8.** Procédé selon la revendication 7, dans lequel le méthane est le produit principal avec une sélectivité de 60 %, tandis que les valeurs de sélectivité pour l'éthane et l'éthylène sont respectivement de 20 à 25 % et de 8 à 10 %.

**9.** Procédé selon la revendication 7 ou 8, dans lequel le rendement global de la production de méthane est de 50 %, tandis qu'il est de 10 à 15 % pour l'éthane et d'environ 10 % pour l'éthylène.

**10.** Utilisation du procédé selon l'une quelconque des revendications précédentes pour réaliser la conversion du $CO_2$ en un mélange de composés chimiques à haute valeur ajoutée ; ce mélange comprenant un mélange d'hydrocarbures de faible poids moléculaire, principalement du méthane, de l'éthylène et de l'éthane, et des produits solides de carbonatation minérale, principalement des carbonates de Mg et de Fe.

Fig. 1

Fig. 2

Fig. 3a) e 3b)

Fig. 4a) e 4b)

Fig. 5

Fig. 6

Fig. 7

Fig. 8)

Fig. 9

Fig. 10

11 A                                    11 B

Fig. 11 A) e 11 B)

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0134538 A1

### Non-patent literature cited in the description

- **R. A. KLEIV et al.** Mechanical activation of olivine. *Miner. Eng.,* 2006, vol. 19, 340-347
- **M. FABIAN et al.** The influence of attrition milling on carbon dioxide sequestration on magnesium-iron silicate. *Miner. Eng.,* 2010, vol. 23, 616-620
- **K. L. SANDVIK et al.** Mechanically activated minerals as a sink for CO. *Adv. Powder Technol.,* 2011, vol. 22, 416-421
- **I. M. POWER et al.** Serpentinite carbonation for CO2 sequestration. *Elements,* 2013, vol. 9, 115-121
- **E. TURIANICOVÁ et al.** A comparison of the reactivity of activated and non-activated olivine with CO. *Int. J. Miner. Process.,* 2013, vol. 123, 73-77
- **J. LI et al.** Ultra-fine grinding and mechanical activation of mine waste rock using a high-speed stirred mill for mineral carbonation. *Int. J. Miner. Metall. Mater.,* 2015, vol. 22, 1005-1016
- **I. RIGOPOULOS et al.** Enhancing the rate of ex situ mineral carbonation in dunites via ball milling. *Adv. Powder Technol.,* 2015, vol. 27, 360-371
- **E. TURIANICOVÁ et al.** A possible way to storage carbon dioxide on mechanically activated olivine (Mg, Fe)2SiO4. *VI Int. Conf. Mechanochemistry Mech. Alloy.,* 2008, 316-319
- **I. RIGOPOULOS et al.** Carbon dioxide storage in olivine basalts: Effect of ball milling process. *Powder Technol.,* 2015, vol. 273, 220-229
- **J. WANGA et al.** *International Ocean and Polar Engineering Conference, Sapporo, Japan,* 10 June 2018
- **M. ROSA et al.** *Journal of CO2 Utilization,* 2015, vol. 9, 82-102
- **A.S. AGARWAL et al.** *ChemSusChem,* 2011, vol. 4, 1301-1310
- Hydrogen in a low-carbon economy. *Committee on Climate Change, London,* November 2018
- **A. ÁLVAREZ et al.** *Chem. Phys. Chem.,* 2017, vol. 18, 3135-3141
- **C. OZE et al.** *Geophysical Research Letters,* 2015, vol. 32, L10203
- **F. WANG et al.** *Minerals Engineering,* 2019, vol. 131, 185-19
- **M. MCCOLLOM et al.** *Geochem.Cosmochim. Acta,* 2001, vol. 65, 3769-3778
- **V. FARINA et al.** *Front. Energy Res.,* 2019, vol. 7, 107
- **E. TURIANICOVÁ et al.** A POSSIBLE WAY TO STORAGE CARBON DIOXIDE ON MECHANICALLY ACTIVATED OLIVINE (Mg,Fe)2SiO4. *INCOME 2008, Jamshedpur, India,* 2008
- **F. TORRE et al.** *Powder Technology,* 2020, vol. 364, 915-923